# FASCICULE DE BREVET EUROPEEN

(11) **EP 1 029 534 B1**
(45) Date de publication et mention de la délivrance du brevet: **31.03.2004**
(21) Numéro de dépôt: 00400184.8
(22) Date de dépôt: 24.01.2000
(51) Int. Cl.: A61K 7/50

(54) **Compositions cosmétiques détergentes contenant un tensioactif hydroxyalkylether anionique et une gomme de guar cationique et leurs utilisations**
Reinigende kosmetische Zusammensetzungen enthaltend anionaktiven Hydroxyalkylethertenside und Guargummi und ihre Anwendung
Cosmetic detergent compositions containing an anionic hydroxyalkyl surfactant and cationic guar gum and their use

(30) Priorité: 16.02.1999 FR 9901867
(43) Date de publication de la demande: 23.08.2000
(73) Titulaire: L'OREAL, 75008 Paris (FR)
(72) Inventeur: Garnier, Nathalie, Springfield, NJ 07081 (US); Cauwet-Martin, Danièle, 75011 Paris (FR); Restle, Serge, 95390 Saint Prix (FR)
(74) Mandataire: Le Blainvaux Bellegarde, Françoise

(56) Documents cités:
- EP-A- 0 291 207
- CHEMICAL ABSTRACTS, vol. 124, no. 14, 1 avril 1996 (1996-04-01) Columbus, Ohio, US; abstract no. 185147, page 622; XP002118900 & JP 07 304653 A (SHISEIDO CO LTD) 21 novembre 1995 (1995-11-21)
- PATENT ABSTRACTS OF JAPAN vol. 013, no. 105 (C-575), 13 mars 1989 (1989-03-13) & JP 63 280008 A (SHISEIDO CO LTD), 17 novembre 1988 (1988-11-17)
- PATENT ABSTRACTS OF JAPAN vol. 017, no. 426 (C-1094), 9 août 1993 (1993-08-09) & JP 05 092914 A (SHISEIDO CO LTD), 16 avril 1993 (1993-04-16)
- PATENT ABSTRACTS OF JAPAN vol. 1996, no. 03, 29 mars 1996 (1996-03-29) & JP 07 304652 A (SHISEIDO CO LTD), 21 novembre 1995 (1995-11-21)

## Description

La présente invention concerne de nouvelles compositions cosmétiques comprenant dans un milieu cosmétiquement acceptable acceptable au moins un tensioactif anionique de type acide hydroxyalkyléther carboxylique et au moins une gomme de galactomannane cationique.

Pour le nettoyage et/ou le lavage des cheveux et/ou de la peau, l'utilisation de compositions détergentes (shampooing ou gel-douche) à base essentiellement d'agents tensioactifs classiques de type notamment anionique, non ionique et/ou amphotère, mais plus particulièrement de type anionique, est courante. Ces compositions sont appliquées sur cheveux ou peau mouillés et la mousse générée par massage ou friction avec les mains permet, après rinçage à l'eau, l'élimination des diverses salissures initialement présentes sur les cheveux ou la peau.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur les matières kératiniques des dommages plus ou moins marqués liés en particulier à l'élimination progressive des lipides ou protéines contenues dans ou à la surface de ces dernières.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

Les agents conditionneurs les plus couramment utilisés à ce jour dans des shampooings sont les polymères cationiques, les silicones et/ou les dérivés siliconés, qui confèrent en effet aux cheveux lavés, secs ou mouillés, une facilité de démêlage, une douceur et un lissage accrus par rapport à ce qui peut être obtenu avec les compositions nettoyantes correspondantes qui en sont exemptes.

Toutefois, et malgré les progrès réalisés récemment dans le domaine des shampooings à base de polymères cationiques et/ou de silicone, ces derniers ne donnent pas vraiment complètement satisfaction, de sorte qu'un fort besoin existe encore actuellement quant à pouvoir disposer de nouveaux produits présentant, au niveau de l'une ou de plusieurs des propriétés cosmétiques évoquées ci-avant, de meilleures performances.

Les tensioactifs anioniques de type acide hydroxyalkyléther carboxylique ont déjà été préconisés dans des compositions cosmétiques détergentes. Ils ont été décrits par exemple dans les demandes de brevet J63280798, J08268487 et J08269482.

Les compositions de lavage des cheveux utilisant ces tensioactifs seuls ne conduisent pas à de propriétés cosmétiques satisfaisantes.

L'invention a donc pour but de proposer des compositions cosmétiques détergentes présentant des propriétés cosmétiques améliorées, en particulier le démêlage, le lissage et la douceur des cheveux.

Or, la demanderesse a maintenant trouvé que l'association de polymères cationiques particuliers et d'un tensioactif anionique de type acide hydroxyalkyléther carboxylique permettait d'atteindre ces buts.

Ces nouvelles compositions permettent de mieux déposer ces polymères cationiques sur les matières kératiniques (notamment les cheveux) qu'une composition contenant des tensioactifs anioniques classiques tels que les sels d'alkyléthercarboxylates, mais sans aspect visuel ou toucher gras.

Les compositions conformes à l'invention confèrent aux matières kératiniques notamment les cheveux, un remarquable effet traitant qui se manifeste notamment par une facilité de démêlage, ainsi qu'un apport de volume, de légèreté, de lissage, de douceur et de souplesse et de discipline sans aucune sensation de toucher chargé.

L'invention a ainsi pour objet une composition cosmétique détergente, caractérisée en ce qu'elle comprend, dans un milieu cosmétiquement acceptable, au moins une gomme de galactomannane cationique et au moins un tensioactif anionique de type acide hydroxy-2 alkyl éthercarboxylique ou ses sels de stucture (I) ci-après.

Un autre objet de l'invention concerne un procédé de traitement des matières kératiniques, telles que les cheveux, caractérisé en ce qu'il consiste à appliquer sur lesdites matières des compositions cosmétiques selon l'invention.

L'invention a encore pour objet l'utilisation de tensioactifs de type acide hydroxy-2 alkyl éther carboxylique dans ou pour la fabrication ce compositions cosmétiques détergentes comprenant au moins une gomme de galactomannane cationique.

Selon la présente invention, par matières kératiniques, on comprend les cheveux, les cils, les sourcils, la peau, les ongles, les muqueuses ou le cuir chevelu et plus particulièrement les cheveux.

Les différents objets de l'invention vont maintenant être détaillés. L'ensemble des significations et définitions des composés utilisés dans la présente invention données ci-dessous sont valables pour l'ensemble des objets de l'invention.

Les tensioactifs anioniques de type acide hydroxy-2 alkylcarboxylique ou ses sels peuvent avoir la structure suivante :

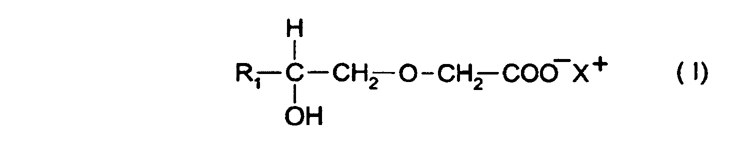

R₁ désigne plus particulièrement un radical alkyle saturé ou insaturé, linéaire ou ramifié comportant de 8 à 30 atomes de carbone.

X désigne l'hydrogène ou un cation minéral ou organique tel que :
ceux issus d'un métal alcalin (par exemple Na⁺, K⁺), NH₄⁺, les ammoniums issus des aminoacides basiques tels que la lysine, l'arginine, la sarcosine, l'ornithine, la citrulline ou bien encore des amino-alcools tels que la monoéthanolamine, la diéthanolamine, la triéthanolamine, la glucamine, la N-méthyl glucamine, l'amino-3 propanediol-1,2.

Des acides hydroxy-2 alkyl éther carboxyliques préférés selon la présente invention sont des composés de formule (I) dans laquelle R₁ désigne plus particulièrement un radical alkyle saturé ou insaturé, linéaire ou ramifié comportant de 8 à 18 atomes de carbone.

Encore plus particulièrement, R₁ désigne un radical un mélange de radicaux en C₈-C₁₈ dérivés de coprah.

Parmi les tensioactifs de formule (I), on peut citer le produit commercialisé sous la dénomination BEAULIGHT SHAA par la société SANYO.

Selon l'invention, le tensioactif anionique de type acide hydroxy-2 alkyl éther carboxylique peut représenter de 1 % à 30 % en poids, de préférence de 3 % à 15 % en poids par rapport au poids total de la composition finale.

Les compositions conformes à l'invention comprennent en outre nécessairement une gomme de galactomannane cationique. De préférence la gomme de galactomannane est une gomme de guar cationique.

Les gommes de galactomannane cationiques ont de préférence une densité de charge cationique inférieure ou égale à 1,5 meq/g et plus particulièrement comprise entre 0,1 et 1 meq./g.

De manière générale, au sens de la présente invention, on entend par "gomme de galactomannane cationique" toute gomme de galactomannane contenant des groupements cationiques et/ou des groupements ionisables en groupements cationiques.

Les groupements cationiques préférés sont choisis parmi ceux comportant des groupements amine primaires, secondaires, tertiaires et/ou quaternaires.

Les gommes de galactomànnane cationiques utilisées ont généralement une masse moléculaire moyenne en poids comprise entre 500 et 5.10⁶ environ, et de préférence comprise entre 10³ et 3.10⁶ environ.

Les gommes de galactomannane cationiques utilisables selon la présente invention sont par exemple des gommes comportant des groupements cationiques trialkyl(C1-C4)ammonium. De préférence, 2 à 30 % en nombre des fonctions hydroxyle de ces gommes porte des groupements cationiques trialkylammonium.

Parmi ces groupements trialkylammonium, on peut tout particulièrement citer les groupements triméthylammonium et triéthylammonium.

Encore plus préférentiellement, ces groupements représentent de 5 à 20 % en poids du poids total de la gomme de galactomannane modifiée.

Selon l'invention, on utilise de préférence une gomme de guar comportant des groupements hydroxypropyl triméthylammonium, c'est à dire une gomme de modifiée guar par exemple par du chlorure de 2,3-époxypropyl triméthylammonium.

Ces gommes de galactomannane en particulier de guar modifiées par des groupements cationiques sont des produits déjà connus en eux-mêmes et sont par exemple décrits dans les brevets US 3 589 578 et US 4 031 307. De tels produits sont par ailleurs vendus notamment sous les dénominations commerciales de JAGUAR C13 S, JAGUAR C 15, JAGUAR C 17 et JAGUAR C162 par la société MEYHALL.

Selon l'invention, la gomme de guar cationique peut représenter de 0,001 % à 10 % en poids, de préférence de 0,005 % à 5 % en poids, et encore plus préférentiellement de 0,01 % à 3 % en poids, du poids total de la composition finale.

Les compositions de l'invention contiennent en outre avantageusement au moins un autre agent tensioactif qui est généralement présent en une quantité comprise entre 0,5% et 40% en poids environ, de préférence entre 3% et 30% et encore plus préférentiellement entre 5% et 20%, par rapport au poids total de la composition.

Cet agent tensioactif peut être choisi parmi les agents tensioactifs anioniques, amphotères, non-ioniques, cationiques ou leurs mélanges.

Les tensioactifs additionnels convenant à la mise en oeuvre de la présente invention sont notamment les suivants :

### (i) Tensioactif(s) anionique(s) :

Leur nature ne revêt pas, dans le cadre de la présente invention, de caractère véritablement critique.
Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s):

Les agents tensioactifs amphotères additionnels, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL, tels que décrits dans les brevets US-2 528 378 et US-2 781 354 et de structures :

R₂-CONHCH₂CH₂-N(R₃)(R₄)(CH₂COO-) (2)

dans laquelle R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₅-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO3H
R₅ désigne un radical alkyle d'un acide carboxylique présent dans l'huile de coprah ou dans l'huile de lin hydrolysée, un radical alkyle, notamment en C₇, C₉, C₁₁, ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Coco-amphodipropionate, Disodium Lauroamphodipropionate, Disodium Caprylamphodipropionate, Disodium Capryloamphodipropionate, Lauroamphodipropionic acid, Coco-amphodipropionic acid.
A titre d'exemple on peut citer le cocoamphodiacetate commercialisé sous la dénomination commerciale MIRANOL® C2M concentré par la société RHODIA CHIMIE.

Dans les compositions conformes à l'invention, on utilise de préférence des mélanges d'agents tensioactifs et en particulier des mélanges d'agents tensioactifs anioniques et des mélanges d'agents tensioactifs anioniques et d'agents tensioactifs amphotères ou non ioniques. Un mélange particulièrement préféré est un mélange constitué d'au moins un agent tensioactif anionique et d'au moins un agent tensioactif amphotère.

On utilise de préférence comme agent tensioactif anionique additionnel les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₄-C₁₆) sulfonate de sodium et leurs mélange avec :
- soit un agent tensioactif amphotère tel que les dérivés d'amine dénommés disodiumcocoamphodipropionate ou sodiumcocoamphopropionate commercialisés notamment par la société RHODIA CHIMIE sous la dénomination commerciale "MIRANOL® C2M CONC" en solution aqueuse à 38 % de matière active ou sous la dénomination MIRANOL® C32;
- soit un agent tensioactif amphotère tel que les alkylbétaïnes en particulier la cocobétaïne commercialisée sous la dénomination "DEHYTON® AB 30" en solution aqueuse à 32 % de MA par la société HENKEL ou tel que les alkyl (C₈-C₂₀) amidoalkyl (C₁-C₆) bétaïnes en particulier la TEGOBETAINE® F 50 commercialisée par la société GOLDSCHMIDT.

Le(s) agent(s) tensioactif(s) anionique(s) différents des acides hydroxy-2alkyléther carboxyliques sont généralement présents à raison de 1 à 30 % en poids, de préférence de 3 à 15 % en poids, par rapport au poids total de la composition.

Le(s) agent(s) tensioactif(s) amphotère(s) ou non ioniques sont généralement présents à raison de 0,5 à environ 15% en poids, de préférence de 1 à 5% en poids, par rapport au poids total de la composition.

La quantité et la qualité des tensioactifs sont celles suffisantes pour conférer à la composition finale un pouvoir moussant et/ou détergent satisfaisant.

Dans la composition selon la présente invention, la totalité des tensioactifs détergents représente généralement de 4 à 50% en poids et de préférence de 6 à 35% en poids et plus particulièrement de 8 à 25% en poids par rapport au poids total de la composition.

On peut également utiliser des tensioactifs cationiques parmi lesquels on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium; les dérivés d'imidazoline ; ou les oxydes d'amines à caractère cationique.

La composition de l'invention peut également contenir au moins un additif choisi parmi les épaississants, les tensioactifs, les parfums, les agents nacrants, les conservateurs, les filtres solaires, les polymères anioniques ou non ioniques ou amphotères, les polymères cationiques différents des gommes de galactomannanes, les protéines, les hydrolysats de protéines, les céramides, les pseudocéramides, les acides gras à chaînes linéaires ou ramifiées en C₁₆-C₄₀ tels que l'acide méthyl-18 eicosanoique, les hydroxyacides, les vitamines, le panthénol, les silicones, les huiles végétales, les huiles minérales et les huiles de synthèse, les agents antipelliculaires et tout autre additif classiquement utilisé dans le domaine cosmétique qui n'affecte pas la stabilité et les propriétés des compositions selon l'invention.

Ces additifs sont présents dans la composition selon l'invention dans des proportions pouvant aller de 0 à 50% en poids par rapport au poids total de la composition. La quantité précise de chaque additif est déterminée facilement par l'homme du métier selon sa nature et sa fonction.

Le milieu cosmétiquement acceptable peut être constitué uniquement par de l'eau ou par un mélange d'eau et d'un solvant cosmétiquement acceptable tel qu'un alcool inférieur en C₁-C₄, comme l'éthanol, l'isopropanol, le tertiobutanol, le n-butanol ; les alkylèneglycols comme le propylèneglycol, les éthers de glycols.
De préférence , la composition comprend de 50 à 95 % en poids d'eau par rapport au poids total de la composition.

Les compositions détergentes selon l'invention présentent un pH final généralement compris entre 3 et 10. De préférence, ce pH est compris entre 4 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide minéral ou organique, de préférence un acide carboxylique tel que par exemple l'acide citrique.

Les compositions conformes à l'invention peuvent contenir en plus de l'association définie ci-dessus des agents régulateurs de viscosité tels que des électrolytes, ou des agents épaississants. On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir jusqu'à 5 % d'agents nacrants ou opacifiants bien connus dans l'état de la technique tels que par exemple les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses tels que par exemple le distéaryléther ou le 1-(hexadécyloxy)-2-octadécanol.

Les compositions selon l'invention peuvent contenir également des synergistes de mousses tels que des 1,2-alcanediols en C₁₀-C₁₈ ou des alcanolamides gras dérivés de mono ou de diéthanolamine.

Les compositions conformes à l'invention peuvent être utilisées pour le lavage et le traitement des matières kératiniques telles que les cheveux, la peau, les cils, les sourcils, les ongles, les lèvres, le cuir chevelu et plus particulièrement les cheveux.

En particulier, les compositions détergentes selon l'invention sont des shampooings, des gels-douche et des bains moussants.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Les compositions de l'invention peuvent encore se présenter sous la forme de produits démaquillants.

Les compositions selon l'invention peuvent se présenter sous forme de gel, de lait, de crème, d'émulsion, de lotion épaissie ou de mousse et être utilisées pour la peau, le cuir chevelu, les ongles, les cils, les lèvres et plus particulièrement les cheveux.

Ces compositions détergentes sont de préférence moussantes et le pouvoir moussant des compositions selon l'invention, caractérisé par une hauteur de mousse, est généralement supérieur à 75 mm ; de préférence, supérieure à 100 mm mesurée selon la méthode ROSS-MILES (NF T 73-404 /ISO696) modifiée.
Les modifications de la méthode sont les suivantes :
La mesure se fait à la température de 22°C avec de l'eau osmosée. La concentration de la solution est de 2g/l. La hauteur de la chute est de 1m. La quantité de composition qui chute est de 200 ml. Ces 200 ml de composition tombe dans une éprouvette ayant un diamètre de 50 mm et contenant 50 ml de la composition à tester. La mesure est faite 5 minutes après l'arrêt de l'écoulement de la composition.

L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage en particulier avec de l'eau.

Ainsi, ce procédé selon l'invention permet le traitement, le soin, le lavage ou le démaquillage de la peau, des cheveux ou de toute autre matière kératinique.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits. Dans les exemples, MA signifie matière active.

### EXEMPLE 1

On a réalisé deux compositions de shampooing , l'une conforme à l'invention et l'autre (B) comparative :

| | A Invention | B Comparatif |
|---|---|---|
| AKYPOSOFT 45 NV de KAO | ― | 15 gMA |
| 2-(2-hydroxylauryloxy)acétate de sodium BEAULIGHT SHAA de SANYO | 15 gMA | ― |
| - Gomme de guar cationique (JAGUAR C13 S de MEYHALL) | 1 g | 1 g |
| Acide citrique qs pH | 7 | 7 |
| Eau déminéralisée qsp | 100 g | 100 g |
| AKYPOSOFT 45 NV (KAO) , Lauryl éther carboxylate de sodium à 4,5 OE en solution aqueuse à 22% de matière active BEAULIGHT SHAA de SANYO 2-(2-hydroxylauryloxy)acétate de sodium en solution aqueuse à 30% de matière active | | |

On effectue un shampooing en appliquant environ 1 g de la composition A ou de la composition B sur des mèches (2, 5 g) de cheveux décolorés préalablement mouillés. On fait mousser le shampooing, on laisse pauser 10 minutes puis on rince abondamment à l'eau courante.

On a ensuite comparé à l'aide d'un test d'évaluation sensorielle, le démêlage à l'état humide des cheveux traités par ces 2 shampooings.

Le test utilisé a pour objet le classement, par un jury, de chaque série de 2 échantillons en attribuant la note 1 pour la mèche qui se démêle le mieux et 2 pour celle qui se démêle le moins bien. Les 2 mèches de la même série sont présentées simultanément au juge. L'analyse statistique des résultats est effectuée à l'aide des tables de A. KRAMER (Food Technology 17 - (12), 124 - 125 1963).

Les 10 testeurs sont unanimes à déclarer que les cheveux lavés avec la composition A sont significativement plus faciles à démêler que ceux traités avec la composition B.

## Revendications

1. Composition cosmétique détergente, **caractérisée par le fait qu'**elle comprend, dans un milieu cosmétiquement acceptable, au moins une gomme de galactomannane cationique et au moins un tensioactif anionique de type acide hydroxy-2 alkylcarboxylique ou ses sels présentant la structure suivante : 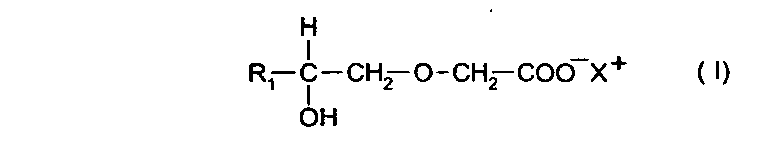
R₁ désigne un radical alkyle saturé ou insaturé, linéaire ou ramifié comportant de 8 à 30 atomes de carbone.
X désigne l'hydrogène ou un cation minéral ou organique tel que ceux issus d'un métal alcalin, NH₄⁺, les ammoniums issus des aminoacides basiques ou des amino-alcools.

2. Composition selon la revendication 1, **caractérisée par le fait que** X désigne Na⁺, K⁺, NH₄⁺, les ammoniums issus de la lysine, l'arginine, la sarcosine, l'ornithine, la citrulline ou issus de la monoéthanolamine, la diéthanolamine, la triéthanolamine, la glucamine, la N-méthyl glucamine ou l'amino-3 propanediol-1,2.

3. Composition selon selon l'une quelconque des revendications 1 ou 2, **caractérisée par le fait que** le radical R₁ désigne un radical alkyle saturé ou insaturé, linéaire ou ramifié comportant de 8 à 18 atomes de carbone.

4. Composition selon l'une quelconque des revendications 1 à 3, **caractérisée par le fait que** le radical R1 est un radical dérivé du coprah.

5. Composition selon l'une quelconque des revendications 1 à 4, **caractérisée par le fait que** ladite gomme de galactomannane cationique comporte des groupements cationiques trialkyl(C1-C4)ammonium.

6. Composition selon l'une quelconque des revendications 1 à 5, **caractérisée par le fait que** ladite gomme de galactomannane comporte des groupements triméthylammonium

7. Composition selon l'une quelconque des revendications 1 à 6, **caractérisée par le fait que** ladite gomme de galactomannane est modifiée par des groupements hydroxypropyl triméthylammonium.

8. Composition selon l'une quelconque des revendications 1 à 7, **caractérisée par le fait que** ladite gomme de galactomannane cationique est une gomme de guar cationique.

9. Composition selon l'une quelconque des revendications 1 à 8, **caractérisée en ce que** le tensioactif anionique de type acide hydroxy-2 alkyl éther carboxylique est présent dans les compositions à une concentration comprise entre 1 et 30 % en poids, de préférence entre 3 et 15% en poids par rapport au poids total de la composition.

10. Composition selon l'une quelconque des revendications 1 à 9, **caractérisée en ce que** ladite gomme de galactomannane cationique est présente à une concentration comprise entre 0,001 % et 10 % en poids par rapport au poids total de la composition, de préférence entre 0,005 % et 5 % en poids et en particulier entre 0,01 % et 3 % en poids.

11. Composition selon l'une quelconque des revendications 1 à 10, **caractérisée par le fait qu'**elle comprend en outre au moins un agent tensioactif additionnel choisi parmi les tensioactifs anioniques, cationiques, non ioniques, amphotères et leurs mélanges.

12. Compositions selon la revendication 11, **caractérisées par le fait que** le ou les agents tensioactifs additionnels sont présents à une concentration comprise entre 0,5% et 40% en poids, de préférence entre 3% et 30% en poids, et encore plus préférentiellement entre 5% et 20% en poids, par rapport au poids total de la composition.

13. Composition selon l'une quelconque des revendications 1 à 12, **caractérisée par le fait qu'**elle comprend en outre au moins un additif choisi parmi les épaississants, les parfums, les agents nacrants, les conservateurs, les filtres solaires, les agents tensioactifs cationiques, les polymères anioniques ou non ioniques ou amphotères, les polymères cationiques différents des gommes de galactomannane, les protéines, les hydrolysats de protéines, les céramides, les pseudocéramides, les acides gras à chaînes linéaires ou ramifiées en C₁₆-C₄₀ tels que l'acide méthyl-18 eicosanoique, les hydroxyacides, les vitamines, le panthénol, les silicones, les huiles végétales, les huiles minérales, les huiles de synthèse.

14. Composition selon l'une quelconque des revendications 1 à 13, **caractérisées par le fait qu'**elle se présente sous forme de shampooing, des gels-douche et des bains moussants, d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

15. Utilisation d'une composition telle que définie dans l'une quelconque des revendications précédentes pour le lavage des matières kératiniques en particulier les cheveux.

16. Procédé de traitement des matières kératiniques, telles que les cheveux, **caractérisé en ce qu'**il consiste à appliquer sur lesdites matières une composition cosmétique selon l'une des revendications 1 à 14, puis à effectuer éventuellement un rinçage.

## Patentansprüche

1. Reinigende kosmetische Zusammensetzung, **dadurch gekennzeichnet, dass** sie in einem kosmetisch akzeptablen Medium mindestens ein kationisches Galactomannan und mindestens einen anionischen grenzflächenaktiven Stoff vom Typ der Carbonsäure-2-hydroxyalkylether oder ihrer Salze der folgenden Struktur enthält. 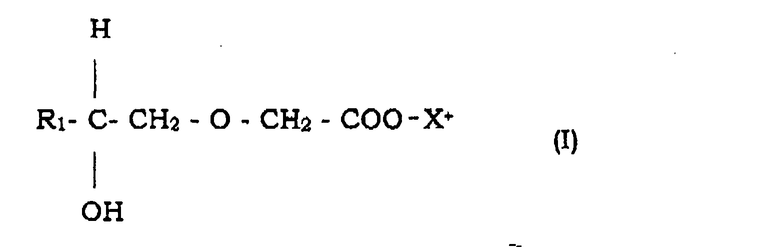 wobei die Gruppe R₁ eine geradkettige oder verzweigte, gesättigte oder ungesättigte Alkylgruppe mit 8 bis 30 Kohlenstoffatomen bedeutet und
X Wasserstoff oder ein anorganisches oder organisches Kation bedeutet, wie z.B. die Kationen, die von einem Alkalimetall stammen, NH₄⁺, Ammoniumionen, die von basischen Aminosäuren oder Aminoalkoholen abgeleitet sind.

2. Zusammensetzung nach Anspruch 1, **dadurch gekennzeichnet, dass** X Na⁺, K⁺, NH₄⁺ oder eine Ammoniumgruppe bedeutet, die von Lysin, Arginin, Sarcosin, Ornithin oder Citrullin abgeleitet ist oder die von Monoethanolamin, Diethanolamin, Triethanolamin, Glucamin, N-Methylglucamin oder 3-Amino-1,2-Propandiol stammt.

3. Zusammensetzung nach einem der Ansprüche 1 oder 2, **dadurch gekennzeichnet, dass** die Gruppe R₁ eine geradkettige oder verzweigte, gesättigte oder ungesättigte Alkylgruppe mit 8 bis 18 Kohlenstoffatomen bedeutet.

4. Zusammensetzung nach einem der Ansprüche 1 bis 3, **dadurch gekennzeichnet, dass** die Gruppe R₁ eine von Kopra abgeleitete Gruppe ist.

5. Zusammensetzung nach einem der Ansprüche 1 bis 4, **dadurch gekennzeichnet, dass** das kationische Galactomannan kationische C₁₋₄-Trialkylammoniumgruppen enthält.

6. Zusammensetzung nach einem der Ansprüche 1 bis 5, **dadurch gekennzeichnet, dass** das Galactomannan Trimethylammoniumgruppen enthält.

7. Zusammensetzung nach einem der Ansprüche 1 bis 6, **dadurch gekennzeichnet, dass** das Galactomannan ein mit Hydroxypropyltrimethylammoniumgruppen modifiziertes Galactomannan ist.

8. Zusammensetzung nach einem der Ansprüche 1 bis 7, **dadurch gekennzeichnet, dass** das kationische Galactomannan ein kationisches Guargummi ist.

9. Zusammensetzung nach einem der Ansprüche 1 bis 8, **dadurch gekennzeichnet, dass** der anionische grenzflächenaktive Stoff vom Typ der Carbonsäure-2-hydroxyalkylether in den Zusammensetzungen in einer Konzentration von 1 bis 30 Gew.-% und vorzugsweise 3 bis 15 Gew.-%, bezogen auf das Gesamtgewicht der Zusammensetzung, enthalten ist.

10. Zusammensetzung nach einem der Ansprüche 1 bis 9, **dadurch gekennzeichnet, dass** das kationische Galactomannan in einer Konzentration von 0,001 bis 10 Gew.-%, vorzugsweise 0,005 bis 5 Gew.-% und insbesondere 0,01 bis 3 Gew.-%, bezogen auf das Gesamtgewicht der Zusammensetzung, enthalten ist.

11. Zusammensetzung nach einem der Ansprüche 1 bis 10, **dadurch gekennzeichnet, dass** sie ferner mindestens einen zusätzlichen grenzflächenaktiven Stoff enthält, der unter den anionischen, kationischen, nichtionischen oder amphoteren grenzflächenaktiven Stoffen und deren Gemischen ausgewählt ist.

12. Zusammensetzung nach Anspruch 11, **dadurch gekennzeichnet, dass** der oder die zusätzliche(n) grenzflächenaktive(n) Stoff(e) in einer Konzentration von 0,5 bis 40 Gew.-%, vorzugsweise 3 bis 30 Gew.-% und noch bevorzugter 5 bis 20 Gew.-%, bezogen auf das Gesamtgewicht der Zusammensetzung, vorliegen.

13. Zusammensetzung nach einem der Ansprüche 1 bis 12, **dadurch gekennzeichnet, dass** sie ferner mindestens einen Zusatzstoff enthält, der unter den Verdickungsmitteln, Parfums, Perlglanzpigmenten, Konservierungsmitteln, Sonnenschutzfiltern, kationischen grenzflächenaktiven Stoffen, anionischen, nichtionischen oder amphoteren Polymeren, kationischen Polymeren, die von den Galactomannanen verschieden sind, Proteinen, Proteinhydrolysaten, Ceramiden, Pseudoceramiden, Fettsäuren mit geraden oder verzweigten C₁₆₋₄₀-Ketten, beispielsweise 18-Methyleicosansäure, Hydroxysäuren, Vitaminen, Panthenol, Siliconen, pflanzlichen Ölen, Mineralölen und synthetischen Ölen ausgewählt ist.

14. Zusammensetzung nach einem der Ansprüche 1 bis 13, **dadurch gekennzeichnet, dass** es sich um ein Haarwaschmittel, ein Duschgel, ein Schaumbad, ein Haarpflegemittel, das ausgespült wird oder im Haar verbleibt, eine Zusammensetzung für permanente Verformungen, Entkräuselungen, Färbungen oder Entfärbungen oder eine Zusammensetzung, die ausgespült wird und vor oder nach einer Färbung, Entfärbung, dauerhaften Verformung oder Entkräuselung oder zwischen den beiden Schritten einer Dauerwelle oder Entkräuselung aufgetragen wird, handelt.

15. Verwendung einer Zusammensetzung nach einem der vorhergehenden Ansprüche zum Waschen von Keratinsubstanzen, insbesondere zum Waschen der Haare.

16. Verfahren zur Behandlung von Keratinsubstanzen, wie dem Haar, **dadurch gekennzeichnet, dass** auf die Keratinsubstanzen eine kosmetische Zusammensetzung nach einem der Ansprüche 1 bis 14 aufgetragen und gegebenenfalls anschließend mit Wasser gespült wird.

## Claims

1. Detergent cosmetic composition, **characterized in that** it comprises, in a cosmetically acceptable medium, at least one cationic galactomannan gum and at least one anionic surfactant of 2-hydroxyalkylcarboxylic acid type, or salts thereof, having the following structure: 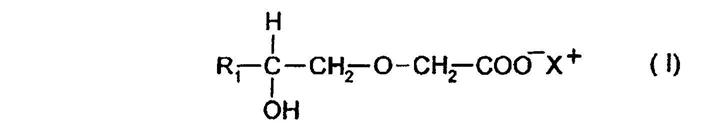
R₁ denotes a saturated or unsaturated, linear or branched alkyl radical comprising from 8 to 30 carbon atoms,
X denotes hydrogen or an inorganic or organic cation such as those derived from an alkali metal, NH₄⁺, the ammoniums derived from basic amino acids or from amino alcohols.

2. Composition according to Claim 1, **characterized in that** X denotes Na⁺, K⁺, NH4⁺, the ammoniums derived from lysine, arginine, sarcosine, ornithine or citrulline or derived from monoethanolamine, diethanolamine, triethanolamine, glucamine, N-methylglucamine or 3-amino-1,2-propanediol.

3. Composition according to either of Claims 1 and 2, **characterized in that** the radical R₁ denotes a saturated or unsaturated, linear or branched alkyl radical comprising from 8 to 18 carbon atoms.

4. Composition according to any one of Claims 1 to 3, **characterized in that** the radical R₁ is a radical derived from copra.

5. Composition according to any one of Claims 1 to 4, **characterized in that** the said cationic galactomannan gum comprises cationic tri(C₁-C₄)alkylammonium groups.

6. Composition according to any one of Claims 1 to 5, **characterized in that** the said galactomannan gum comprises trimethylammonium groups.

7. Composition according to any one of Claims 1 to 6, **characterized in that** the said galactomannan gum is modified with hydroxypropyltrimethylammonium groups.

8. Composition according to any one of Claims 1 to 7, **characterized in that** the said cationic galactomannan gum is a cationic guar gum.

9. Composition according to any one of Claims 1 to 8, **characterized in that** the anionic surfactant of 2-hydroxyalkyl ether carboxylic acid type is present in the compositions in a concentration of between 1 and 30% by weight, preferably between 3 and 15% by weight, relative to the total weight of the composition.

10. Composition according to any one of Claims 1 to 9, **characterized in that** the said cationic galactomannan gum is present in a concentration of between 0.001% and 10% by weight relative to the total weight of the composition, preferably between 0.005% and 5% by weight and in particular between 0.01% and 3% by weight.

11. Composition according to any one of Claims 1 to 10, **characterized in that** it also comprises at least one additional surfactant chosen from anionic, cationic, nonionic and amphoteric surfactants, and mixtures thereof.

12. Composition according to Claim 11, **characterized in that** the additional surfactant(s) is (are) present in a concentration of between 0.5% and 40% by weight, preferably between 3% and 30% by weight and even more preferably between 5% and 20% by weight, relative to the total weight of the composition.

13. Composition according to any one of Claims 1 to 12, **characterized in that** it also comprises at least one additive chosen from thickeners, fragrances, nacres, preserving agents, sunscreens, cationic surfactants, anionic, nonionic or amphoteric polymers, cationic polymers other than galactomannan gums, proteins, protein hydrolysates, ceramides, pseudoceramides, fatty acids containing linear or branched C₁₆-C₄₀ chains, such as 18-methyleicosanoic acid, hydroxy acids, vitamins, panthenol, silicones, plant oils, mineral oils and synthetic oils.

14. Composition according to any one of Claims 1 to 13, **characterized in that** it is in the form of shampoos, shower gels and bubble baths, rinse-out or leave-in conditioners, compositions for permanent-waving, straightening, dyeing or bleaching the hair, or alternatively, in the form of rinse-out compositions to be applied before or after dyeing, bleaching, permanent-waving or straightening the hair or between the two steps of a permanent-waving or hair-straightening operation.

15. Use of a composition as defined in any one of the preceding claims, for washing keratin substances, in particular the hair.

16. Process for treating keratin substances, such as the hair, **characterized in that** it consists in applying a cosmetic composition according to one of Claims 1 to 14 to the said substances, optionally followed by a rinsing operation.
